# EUROPEAN PATENT APPLICATION

(11) **EP 1 183 998 A2**
(43) Date of publication of application: **06.03.2002**
(21) Application number: 01203092.0
(22) Date of filing: 15.08.2001
(51) Int. Cl.: A61B 17/16

(54) **Driver for a rotary surgical tool**

(30) Priority: 30.08.2000 US 651219
(71) Applicant: Grace Manufacturing Inc., Russellville, Arkansas 72801 (US)
(72) Inventor: Grace, Jeff, Dover, AR 72837 (US); Arivett, Joel Dennis, Dover, AR 72837 (US); Grace, Richard Louis, Dover, AR 72837 (US)
(74) Representative: De Hoop, Eric

(57) **Abstract**

A rotary surgical tool driver having a shaft with a chuck end. The chuck end has at least two tongs formed from outside surfaces and inside surfaces which defines a bias cavity. The tongs can be hand biased within the cavity to allow placement of the chuck end of the driver into the opening of the tool. The outside surfaces of the chuck end define various features of the unitary driver that conform to the geometry of the opening in the tool, thus allowing firm engagement between the driver and tool.

## Description

### BACKGROUND OF THE INVENTION

### 1. Field of the Invention:

The present invention relates in general to drivers for surgical tools. More particularly, the present invention relates to a unitary driver for an acetabular reamer having a backing plate with an opening of a geometry that engages the driver.

### 2. Description of the Prior Art:

There are many types of rotary tools used by surgeons to perform various procedures. The most common type of rotary tool is an acetabular reamer used primarily to cut into bone for the implantation of joint prostheses. The most common use of an acetabular reamer is to replace the hip joint, wherein the greater trochanter of a femur and the acetabulum are replaced with a ball and socket-type of prostheses, respectively. In order to perform such operations as to replace the hip joint, the surgeon must ream a portion of the bone and other tissue from the acetabulum to allow placement of the prosthetic socket. The reaming of the bone is accomplished by the use of an acetabular reamer coupled to a driver.

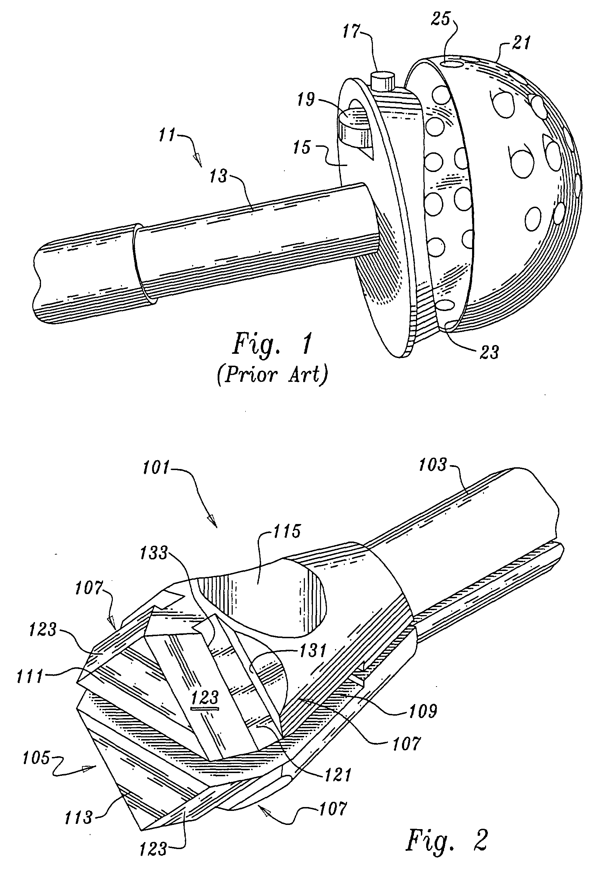

An example of an acetabular reamer and its coupling to a driver is described with respect to **Figure 1,** wherein the driver 11 comprises a shaft 13 coupled to a ratchet plate 15. The plate 15 engages the back edge 23 of acetabular reamer cup 21, while detent 17 engages the detent hole 25. The spring lever 19 controls the detent 17 that holds the driver into the reamer cup 21. This prior art driver has the disadvantage of having moving parts such as the detent, which has a spring mechanism within the ratchet plate 15. This makes cleaning and sterilizing the driver difficult to perform. Further, the design of the driver is such that there are no outlets for cut bone and tissue debris to exit and thus allowing buildup within the reamer cup 21.

There are other drivers for surgical tools in the art. *Salyer* (U.S. Pat. No. 5,980,170) discloses a driver for an acetabular reamer having a boss-controlled latch mechanism using several latch pieces that are moved back and forth to grip the reamer cup. *Salyer* (U.S. Pat. No. 5,817,096) also discloses a driver having a number of small arms that extend radially from a telescopic spring biased rod, the extension of the arms controlled by rotating an arm driver relative to the main shaft. Also, *Lechot* (U.S. Pat. No. 5,658,290) discloses a driver having a slide mounted onto a shaft, the slide having a number of studs for coupling to the reamer cup.

These prior art devices also have the disadvantage of having various moving metal parts in close proximity, thus adding to their mechanical complexity and lack of reliability. Further, these prior art drivers are difficult to clean and sterilize. Sterility is especially important in that during hip-replacement procedures a large portion of the body is exposed, thus necessitating the use of highly sterile devices to make the procedures safe. Most prior art drivers must be disassembled to fully clean and sterilize the various parts, thus making complete sterility difficult to achieve.

Since arthritis is a leading cause for the need to have hip prostheses, it is expected that there will be an increased incidence of hip-replacement surgery and other like surgical procedures. Thus, there is a need for an improved driver that can be used with rotary surgical tools that will simplify surgery and make it safer. The present invention is directed towards such use.

### SUMMARY OF THE INVENTION

It is therefore one object of the present invention to provide an improved surgical tool driver that is simple and reliable to use.

It is another object of the present invention to provide a surgical tool driver that has fewer parts and is thus easier to clean and sterilize.

It is yet another object of the present invention to provide a surgical tool driver that can adapt to various tool geometries and maintain the tool in a stable position.

It is yet another object of the present invention to provide a surgical tool driver that will allow bone and tissue fragments to exit the reamer cup.

These and other objects are achieved by providing a driver for engaging surgical tools having a hollow cavity and an opening with a complex geometry for engaging the driver. The driver comprises a rigid shaft having a distal end and a chuck end, the chuck end having an outer surface forming at least two tongs and an inner surface forming a bias cavity, the entire assembly preferably being of unitary construction. The chuck end comprises contact surfaces on the tongs that are radially configured such that the contact surfaces are tensionally biased against the opening of the surgical tool when engaged with the tool. The tongs are forced closed by the user as they move within the bias cavity. The tongs bias back outward against the tool opening once released by the user. A moveable sleeve is fitted over the shaft that allows the user to control the lateral movement of the shaft while it is being used.

The outer surfaces form various features that facilitate the engagement of the tool to the driver. The two or more tongs terminate in a tapered end and have a slot formed on at least a selected outer surface of the chuck adjacent the tapered end. Each slot also has a proximal lip and a distal lip adjacent to the slot for allowing force to be applied to the driver along its axis.

Additional objects, features and advantages will be apparent in the written description which follows.

### BRIEF DESCRIPTION OF THE DRAWINGS

The novel features believed characteristic of the invention are set forth in the appended claims. The invention itself however, as well as a preferred mode of use, further objects and advantages thereof, will best be understood by reference to the following detailed description of an illustrative embodiment when read in conjunction with the accompanying drawings, wherein:
**Figure 1** is a prior art surgical tool driver and reamer cup;
**Figure 2** is a close-up perspective view of the chuck end of the driver of the invention;
**Figure 3** is a side view of the driver of the invention;
**Figure 4** is a close-up side view of the chuck end of the driver demonstrating the biasing of the tongs;
**Figure 5** is a perspective view of the driver engaged with an acetabular reamer; and
**Figure 6** is an end-on view of another embodiment of the driver of the invention.

### DETAILED DESCRIPTION OF THE INVENTION

The present invention is a driver for engaging surgical tools, especially acetabular reamers, having a hollow cavity and an opening with a complex geometry for engaging a driver in complimentary fashion. Preferably, the opening is associated with a backing plate attached to the reamer. Alternatively, the opening may be part of the reamer cup body that extends to the back of the reamer cup.

The driver comprises a shaft having a distal end and an oppositely extending chuck end, all preferably made from a rigid material and forming one unitary component. A variety of metals and hardened plastics might be utilized to form the driver. Preferably, the driver is formed of surgical steel, stainless steel or titanium. The chuck end has outer surfaces that define various features of the chuck end that act as contact surfaces for engaging the surgical tool and holding it firmly in place on the driver. The inner surfaces define a bias cavity, the bias cavity allowing for radial inward and outward biasing of the outer surfaces of the chuck end. This radial biasing alters the effective diameter of the contact surfaces from an initial diameter to a smaller diameter that will allow the user to place the chuck end of the driver into the surgical tool and engage it.

The outer surfaces of the chuck end comprise contact surfaces radially configured such that the contact surfaces are tensionally biased against the opening of the surgical tool when engaged with the tool. In one embodiment of the driver, the outer surfaces of the chuck further comprise at least two tongs, the bias cavity there between. There can be three or more tongs that comprise the chuck end of the driver, the outer surfaces thereof preferably conforming to the inside geometry of the surgical tool opening. The at least two tongs have at least one slot on each tong defined by the outer surfaces, and the tongs terminate in a tapered end. The slots have a proximal lip and a distal lip to engage the surgical tool and allow the user to apply force upon the surgical tool along the axis of the driver. These contact surfaces are formed to conform to the complex geometry of the surgical tool opening when engaged with the driver.

To facilitate its use, the driver has indents on the outside surfaces to allow the user to close the tongs together with hand pressure to decrease the effective diameter of the contact surfaces and allow the chuck end to be inserted in a tool. Also, a low-friction sleeve is placed on the driver that allows the user to hold the driver and guide and stabilize its lateral movement during use, the sleeve allowing the driver to rotate within. Thus, the user of the driver will typically couple the driver to an electric power drill at its distal end and control the drill with one hand, while holding the driver in the other hand to guide the tool being held by the driver.

The invention is described in greater detail with reference to the figures, and specifically with reference to **Figures 2 - 6.** A close-up view of one embodiment of the chuck end 105 of driver 101 is shown in **Figure 2,** wherein the chuck end is generally defined by having outer surfaces 107 and inner surfaces 109. The chuck end is continuous with a shaft 103. The inside surfaces 109 define a bias cavity, while the outside surfaces 107 define contact surfaces that engage the acetabular reamer backing plate opening. The outside surfaces 107 define the contact surfaces in such a manner as to conform to the geometry of the reamer backing plate opening that it is to be inserted within. This engagement between the backing plate and the contact surfaces maintain a stable and firm grip between the driver 101 and the reamer cup to be driven by the surgeon.

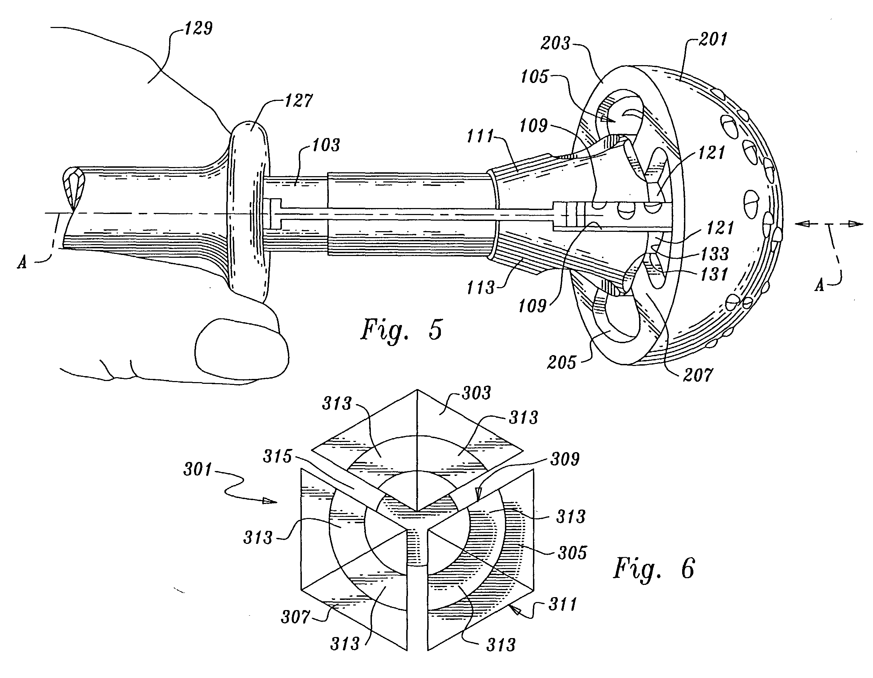

The outside surfaces define tong 111 and 113 in the present embodiment, but can define more than two as shown in **Figure 6.** Referring back to **Figure 2,** the outside surfaces 107 define specific contact surfaces in the embodiment shown in the figures. Slot 121 is defined by the outside surface, the slot having distal lip 131 and proximal lip 133 for engaging the backing plate of the reamer. Each tong 111 and 113 have slots 121 with distal and proximal lips. The outside surfaces 107 also define at least one indent 115. The indent is placed on the chuck end 103 of the driver in order to facilitate the user placing his finger against the chuck. The user then applies pressure for two purposes: to close the tongs 111 and 113 together within the bias cavity, and to insert the closed tongs into the opening of a backing plate. Finally, the tapered ends 123, two on each tong, are defined by the outside surfaces 107 and facilitate the insertion of the driver into the backing plate of the reamer.

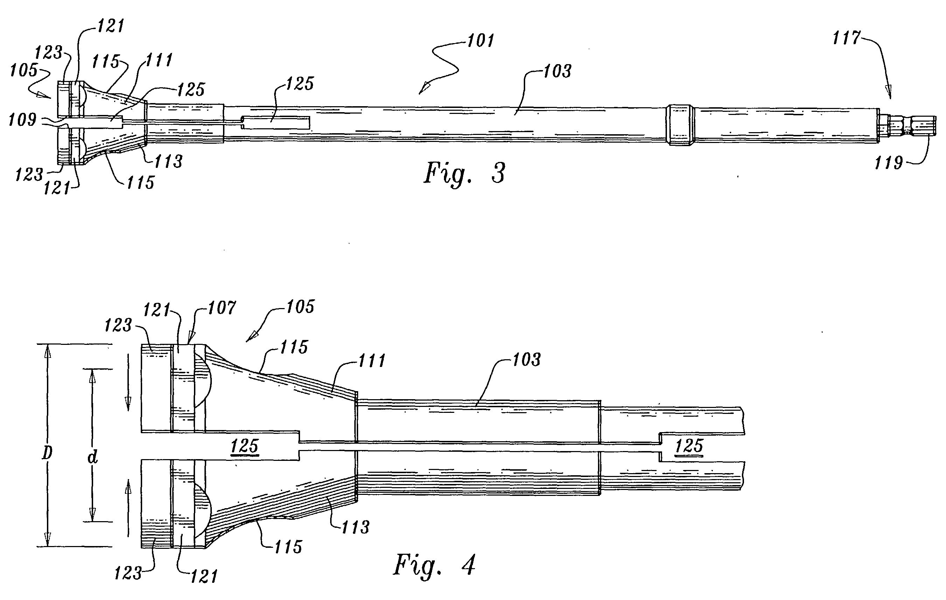

The entire driver 101 is shown in **Figure 3,** wherein the shaft extends to distal end 117 and has coupled to the distal end a drill adaptor 119. The drill adaptor 119 is used to couple the driver with a mechanical or electrical drill or other apparatus operated by the surgeon during an operation such as in hip-replacement surgery. The bias cavity 125 is shown in this embodiment as being an elongated space between tongs 111 and 113, the tongs extending from the driver as a uniform part thereof to form the chuck end 105. The inner surfaces 109 defining the bias cavity 125 can be of any shape and dimension that will allow the user to bias the two or more tongs together for insertion into the acetabular reamer backing plate. The outside surfaces define the slots 121, and tapered ends 123. Each tong has at least two tapered ends 123 in the present embodiment in order to facilitate the insertion of the chuck end 105 of the driver into the backing plate to be used.

The insertion of the driver into the backing plate of an acetabular reamer is shown with respect to **Figures 4** and **5,** wherein the arrows in **Figure 4** indicate the closing of the tongs 111 and 113 with respect to one another within the bias cavity 125. The user, using a hand, can close the two tongs 111 and 113 together with finger pressure to decrease the distance between the contact surfaces and hence the effective diameter D defined by the opened, biased tongs to a closed position of diameter d. The closing of the tongs to a smaller diameter d relative to the resting diameter D allows the slots 121 to be inserted into the backing plate. The slots are effectively recessed into the tongs 111 and 113, their depth defined by the distal 131 and proximal 133 lips.

When the diameter of the outside surfaces 107 is defined by diameter d, the driver is pushed or placed into the opening of a backing plate as shown with respect to **Figure 5.** The driver in **Figure 5** is engaged with acetabular reamer cup 201 having a backing plate 203 and complex profile geometry 205 which define arms 207. The driver is inserted such that the backing plate arms 207 engage the slots 121. The user then releases pressure from the tongs and allows the tongs to bias into their resting position back to the diameter D so that the slots 121 engage the backing plate arms 207. The four arms 207 of backing plate 203 will engage the lips 131 and 133 of each slot if any force is placed upon the driver along axis A, the force expressed by the arrows in **Figure 5** along the axis A.

A user can control the lateral movement of the driver while in use by gripping a sleeve 127 with a hand 129. The sleeve is fitted around the driver shaft 103 in such a manner as to allow a small amount of movement between the two. Further, the sleeve is preferably made from a low-friction plastic or polymeric material such as Teflon. Thus, when the driver is coupled to a drill or other device operated by the surgeon, the user can grip the driver using the sleeve 127 while the driver is being turned about the axis A.

Another embodiment of the driver is shown with respect to **Figure 6,** wherein driver 301 is shown chuck end-on having three tongs 303, 305, and 307. The outside surfaces 311 define the slots 313 and other features, while the inside surfaces 309 define the bias cavity 315. The outside surfaces 311 define a surface that will conform to the complex geometry of the opening of an acetabular reamer. In the embodiment of **Figure 6,** the backing plate ideally will have six arms to engage each of the six slots of driver 301.

The present invention offers several advantages over the prior art. The present driver is a unitary component, thus easy to clean and completely sterilize. The driver is simple to use since its initial alignment with the reamer cup is not critical. The driver, once engaged with the cup, self-aligns. The driver is more reliable than prior art drivers since it is a unitary design. Further, the sleeve can be easily removed for cleaning since it can be slid on or off the distal end of the driver.

While the invention has been shown in only one of its forms, it is not thus limited but is susceptible to various changes and modifications without departing from the spirit thereof.

## Claims

1. A driver for engaging surgical tools having a hollow cavity and an opening with a complex geometry for engaging a driver, the driver comprising:
a shaft having a distal end and an oppositely extending chuck end having outer surfaces and having inner surfaces, the inner surfaces defining a bias cavity therein, the bias cavity allowing for radial inward and outward biasing of the outer surfaces of the chuck end; and
wherein the outer surfaces of the chuck end comprise contact surfaces radially configured such that the contact surfaces are tensionally biased against the opening of the surgical tool when engaged with the tool.

2. The driver of Claim 1, wherein outer surfaces of the chuck further comprise at least two tongs, the bias cavity there between.

3. The driver of Claim 2, wherein the at least two tongs terminate in a tapered end.

4. The driver of Claim 3, wherein a slot is formed on at least a selected outer surface of the chuck adjacent the tapered end.

5. The driver of Claim 4, further comprising a proximal lip and a distal lip adjacent to each slot.

6. The driver according to any one of the preceding claims, wherein the contact surfaces are formed to conform to the complex geometry of the surgical tool opening when engaged.

7. The driver according to any one of the preceding claims, wherein the outer surface of the chuck end has at least one indent to allow a finger to apply pressure against the chuck end.

8. A driver for engaging an acetabular reamer having a hollow cavity and an outer edge coupled to a backing plate, the backing plate having an opening with a complex geometry for engaging the driver, the driver comprising:
a shaft having a distal end and an oppositely extending chuck end having outer surfaces and having inner surfaces, the inner surfaces forming a bias cavity;
wherein the outer surfaces of the chuck end comprise at least two tongs having the bias cavity there between; and
wherein a force applied against the at least two tongs biases the tongs in an engaging position with respect to the opening of the reamer backing plate, thus engaging the outer surfaces with the acetabular reamer backing plate opening.

9. The driver of Claim 8, wherein the at least two tongs terminate in a tapered end.

10. The driver of Claim 8 or 9, wherein a slot is formed in at least a selected outer surface of the chuck adjacent the tapered end.

11. The driver of Claim 8, 9 or 10, wherein a slot is formed on an outer surface of each tong and wherein each slot on the at least two tongs collectively comprise contact surfaces for tensionally engaging the reamer backing plate.

12. The driver of any one of the preceding claims 8-11, further comprising a proximal lip and a distal lip adjacent to the slot.

13. The driver according to any one of the preceding claims 8-12, wherein the contact surfaces are formed to conform to the complex geometry of the reamer backing plate opening when engaged.

14. The driver according to any one of the preceding claims 8-13, wherein the outer surface of the chuck end has at least one indent to allow a finger to apply pressure against the chuck end.

15. A method for engaging a driver with a surgical tool having a hollow cavity and an opening with a complex geometry, the method comprising:
providing a rigid shaft having a distal end and a chuck end, the chuck end having an outer surface forming at least two tongs and an inner surface forming a bias cavity;
wherein the chuck end comprises contact surfaces radially configured such that the contact surfaces are tensionally biased against the opening of the surgical tool when engaged with the tool; and
wherein the two tongs of the chuck end are biased inwardly by hand pressure of a user to thereby install the chuck end within the opening of the surgical tool, and wherein removing the hand pressure of the user allows outward radial movement of the two tongs of the chuck end to thereby engage the contact surface with the openings of the surgical tool.

16. The method of claim 15, wherein the driver is removed from the surgical tool by first inwardly biasing the two tongs and then withdrawing the chuck end of the driver from the opening in the surgical tool.

17. The method of Claim 15 or 16, wherein the contact zones are formed to conform to the complex geometry of the surgical tool opening when engaged.

18. The method of Claim 15, 16 or 17, wherein the outer surface of the chuck end has an indent to allow a finger to apply pressure against the chuck end.

19. The method according to any one of the preceding claims 15-18, wherein the shaft of the driver is received within a surrounding sleeve during use, the sleeve allowing rotation of the shaft to, in turn, rotate the engaged surgical tool.
